## Europäisches Patentamt

## European Patent Office

## Office européen des brevets

(19)

(11) **EP 1 522 288 A2**

(12) # EUROPEAN PATENT APPLICATION

(43) Date of publication:
**13.04.2005 Bulletin 2005/15**

(51) Int Cl.⁷: **A61F 9/00**

(21) Application number: **05075014.0**

(22) Date of filing: **20.12.1996**

(84) Designated Contracting States:
**AT BE CH DE DK ES FI FR GB GR IE IT LI LU MC NL PT SE**

(30) Priority: **21.12.1995 GB 9526150**

(62) Document number(s) of the earlier application(s) in accordance with Art. 76 EPC:
**96943216.0 / 0 873 096**

(71) Applicant: **Pfizer Health AB**
**112 87 Stockholm (SE)**

(72) Inventors:
• **Embleton, Jonathan Kenneth**
**Chesterton Lane Cirencester GL7 1WJ (GB)**

• **Malcolmson, Richard Joseph**
**Freshbrook Swindon SN5 8RA (GB)**
• **Martini, Luigi Gerard Anthony**
**Welwyn Garden City Herts AL7 3JW (GB)**

(74) Representative: **Hitchcock, Esmond Antony**
**Lloyd Wise**
**Commonwealth House,**
**1-19 New Oxford Street**
**London WC1A 1LW (GB)**

Remarks:
This application was filed on 05 - 01 - 2005 as a divisional application to the application mentioned under INID code 62.

(54) **Ophthalmic treatment**

(57)     The bioavailability of an ophthalmologically active compound is increased by its provision in a dosage of ophthalmic treatment liquid which takes the form of a jet or stream of droplets. Such a jet or stream can be directed or targeted at a particular site in the eye where the compound can be best absorbed.

**EP 1 522 288 A2**

**Description**

This invention relates to ophthalmic treatment and more particularly, to dosage forms useful in such treatment. The invention is concerned only with liquid treatment substances.

Ocular medication is most frequently administered as eye drop solutions. The typical volume of an eye drop has been found to range from 25 μl to 50 μl. Under normal conditions, in the open eye the human tear volume remains relatively constant at around 7 μl, with continuous drainage of tear fluid (via the nasolacrimal canal) being replaced by the tear glands. The tear volume can increase to about 30 μl before overflowing occurs and the excess fluid is lost either through the nasolacrimal duct or by spillage onto the cheek. Blinking reduces this maximal volume to say, 10 μl. Thus the addition of large volumes of liquid such as those presented in commercial eyedrops will result in the rapid elimination of the active agents from the eye with typically 80-90 % of an instilled drop being lost within one minute. Drug which drained through the highly vascular nasolacrimal duct can be absorbed into the systemic circulation as a bolus dose and therefore by-pass hepatic metabolism.

The recent use of β-blocking agents in ophthalmology has highlighted the disadvantages associated with this rapid drainage process, with serious life threatening side-effects such as bradycardia, bronchospasm and even heart failure being induced in susceptible patients. In addition, research has also shown that the rate at which instilled solutions are drained from the eye varies directly with the instilled volume i.e. the larger the instilled volume, the more rapidly it is removed from the precorneal regions of the eye. These findings have led to the suggestion that a higher concentration of drug in as small a volume as is practicable would be beneficial. In one study published in the American Journal of Ophthalmology 85, 1978 pp 225 to 229; Ocular bioavailability and systematic loss of topically applied ophthalmic drugs, by Thomas Patton and Michael Francoeur, it was reported that when using a 5 μl eye drop loaded with 26.1 μg of pilocarpine nitrate, the fraction of drug absorbed into the eye was 0.41 μg, leaving 25.7 μg available for potential systemic absorption. A similar calculation using a 25 μl drop loaded with 67.8 μg of pilocarpine nitrate, revealed that 0.36 μg had penetrated the eye, thus leaving 67.4 μg to be absorbed systemically. From this kind of study it can be concluded:

1. That an argument could be made for the use of smaller instilled volumes of eye drops than are normally delivered by most commercial ophthalmic droppers. Drainage loss would be minimised; contact time increased and hence the potential exists for improved drug activity.
2. Due to reduced drainage, less total volume of eye drop solution, and hence less drug need be used, therefore reducing the risk of systemic side-effects, whilst improving cost efficiency due to less wastage.

The research work referred to above is restricted to the use of ophthalmic solutions delivered as instillates. Surprisingly, we have found that the ocular bioavailability of ophthalmologically active compounds can be further enhanced by delivery to the eye in the form of a jet or stream of droplets. Particularly, we have found that smaller quantities of the same treatment liquid, when delivered in this manner can have the same or an improved pharmacological effect. Accordingly, the present invention provides a dosage form useful in ophthalmic treatment, comprising a jet or stream of droplets of treatment fluid, the jet or each droplet having an ophthalmologically active compound in suspension or solution, normally an aqueous solution. The jet or stream can be directed or targeted at a chosen site in an eye; eg, cornea, anterior bulbar conjunctiva, posterior bulbar conjunctiva or palpebral conjunctiva where the active compound can be most readily absorbed.

While dosage forms according to the invention can be delivered vertically, under the force of gravity, preferred forms are also suitable for horizontal delivery. In such forms, the jet or each droplet is of a size sufficient to sustain its momentum in transmission from a delivery device to a target site. Preferably, the size of the jet or each droplet is sufficient to sustain its momentum along a substantially horizontal path of 5 cm in length from a discharge velocity of up to 25 m/sec from a delivery device. A typical minimum discharge velocity is 5 m/s. As a general guide jet/droplet diameters in the range 20 to 1000 μm are suitable in the practice of the invention. A typical mean diameter for these purposes is in the range 100 to 800 μm, preferably 200 to 400 μm. This narrower range is a preferred guide, and in practice may not be critical. The efficacy of this invention is not adversely affected if the mean diameter is outside of this limit.

The enhanced bioavailability of ophthalmologically active compounds in dosage forms according to the invention enables the use of even smaller total volumes of treatment fluid than proposed in the eye drop study discussed above. Typically, the total volume of treatment fluid in a dosage form according to the invention does not exceed 20 μl, preferably no greater than 10 μl, and most preferably, in the range 3 to 8 μl. The discharge of such a small volume from a delivery device at a suitable velocity to create the jet or stream will normally beat the "blink response" and result in a high percentage of the active compound in the treatment fluid performing its intended function. In other words, the entire volume can be delivered to the chosen site on the eye before the patient blinks to disperse the received fluid.

Treatment fluid used in dosage forms of the invention can additionally contain excipients to prolong the residence time in the cul-de-sac (the conjunctival sac), and thereby further enhance bioavailability. Suitable excipients include viscosity modulators, polymers, gelling agents and thickeners.

The invention will now be described with reference to the following examples.

Example 1 EPHEDRINE

Six white New Zealand rabbits were administered with the following dosage regimen:

I → 25 µl of 1 % aqueous ephedrine hydrochloride solution (250 µg) via pipette (instillate)
II → 5 µl of 5 % aqueous ephedrine hydrochloride solution (250 µg) via pipette (instillate)
III → 5 µl of 5 % aqueous ephedrine hydrochloride solution (250 µg) in a jet/stream of droplets of diameter in the range 200 to 400 µm.

Pupil diameter measurements were determined from photographs acquired using a Pentax ME super 35 mm camera fitted with a SMC Pentax 50 mm lens and a 2x converter. An aperture setting of 12, and a shutter speed of 1/15 was employed with a film speed of ISO 400 (Kodak Gold 400). The camera was held stationary on a tripod and positioned approximately 30-40 cm from the rabbits eye. Prior to each dosing period the animals were acclimatised to experimental conditions (constant light intensity, minimal distractions) for 20 min. The rabbits were placed in restraining boxes and settled before photographs and baseline pupil diameters were determined 5 min prior to dosing.

Pupillary diameters were determined from the developed colour prints (6 x 4) using an electronic micrometer (Digimatic Caliper, Mitutoyo Corp., Japan). Absolute pupil diameters were established by comparing the pupil diameter with a scale of known magnitude placed next to and in the same plane as the pupil prior to photography. The maximum response ratio ($RR_{max}$) for pupil dilation was then calculated from the photographs using the following relationship:

$$(RR_{max}) = (\text{pupil diameter time t - average pupil diameter}$$

$$\text{time 0}) / \text{average pupil diameter time 0}.$$

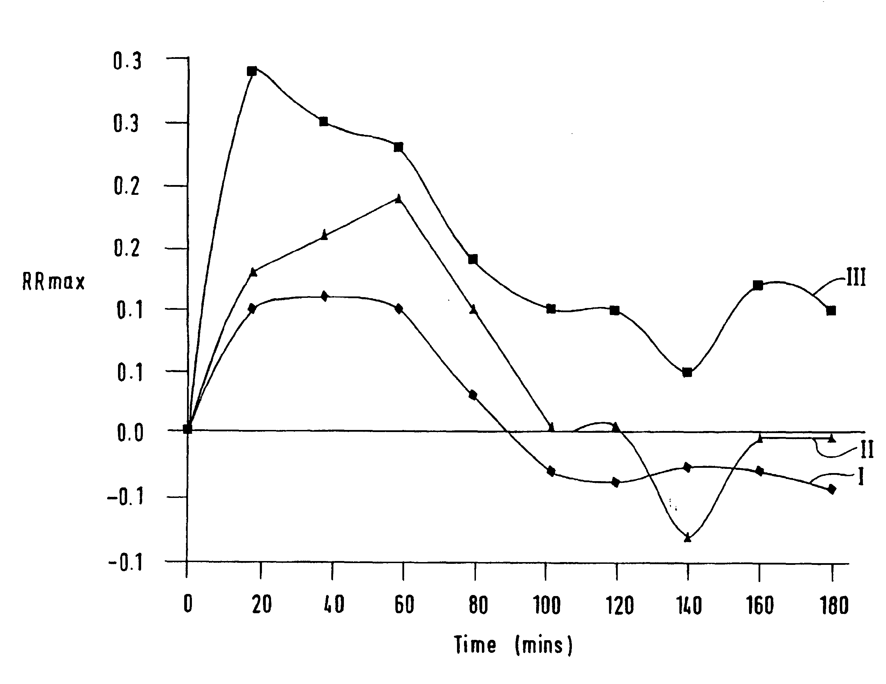

The graph of Figure 1 was then plotted of mean values of $RR_{max}$ against time. Curves I, II, and III represent the results from use of the respective dosage regimen referred to above.

Results

It can be seen from Figure 1 that the mydriatic response obtained from the 5 µl ocular droplet dosage form was more pronounced and maintained over a longer duration compared to both instillates; in terms of $RR_{max}$ values the response can be ranked as follows : 5 µl ocular droplet stream > 5 µl instillate > 25 µl instillate.

Instillates are normally administered directly into the conjunctival sac with reflex blinking distributing the majority of the solution over the cornea. Even with small volume instillates, a substantial proportion of the solution is still emptied directly into the nasolacrimal drainage system. In using dosage forms of the invention targeted directly at the cornea our results showed that the solution uniformly covered the cornea with minimal splash-back upon impact, with a gradual pooling of liquid towards the conjunctival sac. Blinking in these instances distributed the solution over the corneal surface even further. This comparative study clearly shows that small volume ophthalmic solutions delivered in a droplet stream enhanced the bioavailability of ephedrine in comparison to the instillate presented from many commercial eyedroppers. A similar effect would be expected using other ophthalmic drugs.

Example 2 PILOCARPINE HCl

Ten white New Zealand rabbits were treated with the following dosage regimen in a randomised cross-over study:

30 µl of 1 % aqueous pilocarpine hydrochloride solution (300 µg) was instilled via pipette into the conjunctival sac
5 µl of 1 % aqueous pilocarpine hydrochloride solution (50 µg) was applied as a jet and/or stream of droplets (with a diameter in the range of 200 µm to 400 µm) to the surface of the cornea.

In order to determine pupil diameters, a metallic rule with a circular aperture of known diameter was orientated perpendicular to, and at an appropriate fixed distance from, a video camera fitted with a macro lens (Sony V8 Pro-CDD-V100E) throughout the study. During miotic measurements, the animals were positioned such that the left eye was parallel to the ruler and equidistant from the video camera. The video camera was actuated to project and amplify images of both the reference aperture and left eye onto the monitor screen. The diameters of both the reference aperture and pupil were then measured on the screen using a ruler placed on the projected image at an angle of approximately 135-305 degrees. The value of the pupil diameter was then calculated by multiplying the projected screen pupil diameter by the ratio of the actual reference diameter (8 mm) to the projected screen reference diameter (18 mm).

Pupil measurements were taken at approximately 60, 45, 30 and 15 minute intervals prior to administration of the treatments to provide a baseline value, and then at 15 minute intervals for the first hour after dosing. Thereafter, the pupil diameter was measured at 30 minute intervals for a minimum duration of 4 hours after dose administration.

For the purpose of statistical analysis of variants between treatments, the following parameters were determined: RR max = (pupil diameter at time t - pupil diameter at time 0) / pupil diameter at time 0; T max = the first time point at which the smallest pupillary diameter was observed; and AUC (0-4 hours) = the area under the pupillary diameter vs. time curve between 0 and 4 hours after treatment.

All significance tests were two-tailed and were performed at the 5 % significance level. The statistical software SAS V607 and the PROC GLM procedure were used in the analysis.

Results

Pupil diameter measurements were taken over the time course of the experiments. Some variation in the pupil diameter could be seen in the predose data, with a significant (P=0.0001) decrease in mean diameter being observed for both treatments as a function of time. The Shapiro-Wilk test for normality revealed that the errors associated with the pupil diameter readings were independently and normally distributed. Pupil diameter measurements were also taken following pilocarpine administration. A reduction in pupil diameter was evident for both dose forms after 15 minutes. However, this effect started to disappear approximately 60-90 minutes after treatment and, after 120 minutes, the measurements had fully recovered to their predose levels.

| Pilocarpine Treatment | AUC (0-4hours) mmMin. | T max Min. | RR max % |
|---|---|---|---|
| 1 % 30 µl large eyedrop (300 µg) | $3871 \pm 340^*$ | $25.5 \pm 12.5^*$ | $15.2 \pm 4.0^*$ |
| 1 % 5 µl jet and/orstream of droplets (50 µg) | $3827 \pm 312^*$ | $24.0 \pm 23.7^*$ | $12.3 \pm 5.2^*$ |

* standard deviations of the mean

The table compares the two treatments in terms of their effects on RR max, T max and AUC. There was no statistically significant difference in the calculated values of RR max, T max or AUC between either of the treatments. Thus, this work demonstrates that an ophthalmic dosage form comprised of a jet and/or stream of droplets can produce an equivalent pharmacodynamic effect to a standard eyedrop with only 1/6 of the drug.

Example 3 PROPRANOLOL HCl (Ocular distribution study)

40 µl of 0.5 % aqueous tritiated propranolol hydrochloride solution (200 µg) was administered via pipette into the conjunctival sac of twelve New Zealand white rabbit eyes. Seperately, 5 µl of 4 % aqueous tritiated propranolol hydrochloride solution (200 µg) was applied as a jet or stream of droplets (with a diameter in the range of 200 µm to 400 µm) to the surface (cornea and/or conjunctiva) of twelve different New Zealand white rabbit eyes.

Following each treatment, four eyes were ennucleated after 15 minutes, four after 30 minutes and the remaining four after 60 minutes. In each case, the animals were humanely killed prior to the ocular ennucleation procedure via an overdose of sodium pentobarbitol injected into a marginal ear vein. Each eye was then irrigated by instilling 100 µl of normal saline into the conjunctival sac using an automatic pipette and immediately blotting away excess saline with paper tissue to remove any radioactivity in the tear film. Following ennucleation and removal of the adnexal tissue, the cornea was washed with a second 100 µl of normal saline. The aqueous humor was then quickly removed by paracentesis with a 1 ml syringe and 26G needle. To this was then added an equal volume of trichloroacetic acid (TCA) solution (10 % w/v) to bring the final concentration to 5 % w/v TCA. Both eyes were then dissected from the posterior pole to allow removal of the vitreous humor and the lens, whilst the iris-cilary body was transferred to a tared sample tube. The cornea was then removed using a 12 mm trephine, and the limbal cornea and conjunctiva (with underlying sclera) cut free into a strip approximately 5 mm wide using a scalpel and scissors. Each sample was then weighed in a tared sample tube and at least 5 volumes of TCA (6 % w/v) added. All tissue samples were subjected to sonication for 5 minutes, then centrifuged at 10 000 g.min. to yield a supernatent. Each supernatent was then extracted 3 times with 3 volumes of ether and, after evaporation of residual solvent, the aqueous residue was sampled and added to 5 ml of FluoronSafe XE "Scintron" scintillation fluid (BDH Chemicals, UK). Radioactivity was then determined by counting in a Packard 1600DR beta-scintillation counter. Data gathered as counts per minute was then converted into disintegrations per minute (dpm), using external standardisation, and expressed as dpm per g of tissue after adjusting for the total radioactivity in each dose. Due to the small number of samples per time point per treatment, statistical analysis was not considered appropriate for this study.

Results

The results of this study are summarised for the different ocular tissues in tables 1 to 4 below, where the values shown represent dpm (disintegrations per minute) per mg of tissue.

Table 1:

| Cornea | | | |
|---|---|---|---|
| Propranolol Treatment | 15 mins | 30 mins | 60 mins |
| 0.5 % 40 µl large eyedrop (200 µg) | 5579 | 3467 | 2945 |
| 4.0 % 5 µl jet and/or stream of droplets (200 µg) | 5241 | 3766 | 1861 |

Table 2:

| Conjunctiva/sclera | | | |
|---|---|---|---|
| Propranolol Treatment | 15 mins | 30 mins | 60 mins |
| 0.5 % 40 µl large eyedrop (200 µg) | 2569 | 2838 | 1380 |
| 4.0 % 5 µl jet and/or stream of droplets (200 µg) | 5286 | 2259 | 1673 |

Table 3:

| Aqueous humor | | | |
|---|---|---|---|
| Propranolol Treatment | 15 mins | 30 mins | 60 mins |
| 0.5 % 40 µl large eyedrop (200 µg) | 1310 | 960 | 705 |
| 4.0 % 5 µl jet and/or stream of droplets (200 µg) | 1845 | 1176 | 607 |

Table 4:

| Iris-cilary body | | | |
|---|---|---|---|
| Propranolol Treatment | 15 mins | 30 mins | 60 mins |
| 0.5 % 40 µl large eyedrop (200 µg) | 942 | 1033 | 799 |
| 4.0 % 5 µl jet and/or stream of droplets (200 µg) | 2256 | 1482 | 586 |

Significant radioactivity was detected in all ocular tissues at all time points for both treatments.

Following dose administration the drug will initially be absorbed into either the cornea or conjunctiva. It would then be expected to partition into the aqueous humor and finally reach the iris-cilary body, which is the site of action for an ophthalmic beta blocker. The concentration of drug in this tissue is therefore of paramount importance in terms of clinical efficacy, i.e. intra-ocular pressure (IOP) reduction. Moreover, recent literature reports (ref: S.A. Sadiq and S. A. Vernon, British Journal of Ophthalmology. 1996 Vol. 80, pp. 532-535) with the most widely used ophthalmic beta blocker, timolol maleate, suggest that the rate at which drug saturates the ocular beta-adrenoceptors in the iris-cilary body is also of considerable importance in terms of clinical efficacy. The rationale here is that rapid heavy blockade of the receptor sites maximises inhibition of aqueous humor secretion and, therefore, IOP reduction.

This fact is of considerable importance when interpreting the results from the present study. Thus, the level of propranolol reaching the iris-ciliary body early (i.e. at the 15 minute time point) from the jet and/or stream of droplets was more than double that obtained from the eyedrop. Such a rapid and substantial accumulation of the beta blocker at its target site would be expected to produce a marked benefit in terms of beta-adrenoceptor inhibition and, therefore, IOP reduction. The comparatively higher level of radioactivity in the iris-cilary body from the eyedrop after 60 minutes probably reflected re-absorption from the local vasculature.

The concentrations of propranolol in the other tissues are not directly relevant from a therapeutic viewpoint, as the iris-cilary body is the only site of aqueous humor formation in the eye. Therefore, although the concentrations of propranolol in some of these other tissues are higher at certain timepoints from the eyedrop compared to the other dosage form, this is unlikely to be of direct relevance to the levels of beta-adrenoceptor inhibition and, therefore, the suppression of aqueous humor formation.

Ophthalmic treatment liquids that may be used with the invention may be aqueous or non-aqueous liquids, optionally containing a therapeutic compound or compounds such as:

1) Anti-glaucoma/IOP (intra-ocular pressure) lowering compounds

    a) β-adrenoceptor antagonists, e.g. carteolol, cetamolol, betaxolol, levobunolol, metipranolol, timolol, etc.
    b) Miotics, e.g. pilocarpine, carbachol, physostigmine, etc.
    c) Sympathomimetics, e.g. adrenaline, dipivefrine, etc.
    d) Carbonic anhydrase inhibitors, e.g. acetazolamide, dorzolamide, etc.
    e) Prostaglandins, e.g. PGF-2 alpha and derivatives thereof such as latanoprost.

2) Anti-microbial compounds (including anti-bacterials and anti-fungals), e.g. chloramphenicol, chlortetracycline, ciprofloxacin, framycetin, fusidic acid, gentamicin, neomycin, norfloxacin, ofloxacin, polymyxin, propamidine, tetracycline, tobramycin, quinolines, etc.

3) Anti-viral compounds, e.g. acyclovir, cidofovir, idoxuridine, interferons, etc.

4) Aldose reductase inhibitors, e.g. tolrestat, etc.

5) Anti-inflammatory and/or anti-allergy compounds, e.g. steroidal compounds such as betamethasone, clobetasone, dexamethasone, fluorometholone, hydrocortisone, prednisolone etc. and non-steroidal compounds such as antazoline, bromfenac, diclofenac, indomethacin, lodoxamide, saprofen, sodium cromoglycate, etc.

6) Artificial tear/dry eye therapies, comfort drops, irrigation fluids, etc., e.g. physiological saline, water, or oils; all optionally containing polymeric compounds such as acetylcysteine, hydroxyethylcellulose, hydroxymellose, hyaluronic acid, polyvinyl alcohol, polyacrylic acid derivatives, etc.

7) Diagnostics, e.g. fluorescein, rose bengal, etc.

8) Local anaesthetics, e.g. amethocaine, lignocaine, oxbuprocaine, proxymetacaine, etc.

9) Compounds which assist healing of corneal surface defects, e.g. cyclosporine, diclofenac, urogastrone and growth factors such as epidermal growth factor, etc.

10) Mydriatics and cycloplegics e.g. atropine, cyclopentolate, homatropine, hysocine, tropicamide, etc.

11) Compounds for the treatment of pterygium, such as mitomycin C, collagenase inhibitors (e.g. batimastat) etc.

12) Compounds for the treatment of macular degeneration and/or diabetic retinopathy and/or cataract prevention.

13) Compounds for systemic effects following absorption into the bloodstream after ocular administration, e.g. insulin.

The above compounds may be in the form of free acids or bases or alternately as salts of these. Combinations of compounds e.g. an anti-bacterial combined with an anti-flammatory may be desirable for the optimization of therapy in some instances. The compounds may be formulated as aqueous or non-aqueous (e.g. oil) solutions or suspensions. Formulations may optionally contain other formulation excipients, for example, thickening agents such as gels, mucoadhesives and polymers, stabilisers, anti-oxidants, preservatives, pH/tonicity adjusters etc.

Devices suitable for delivering dosage forms in accordance with the present invention are described in our International Patent Application Nos. GB95/01482 and GB95/02040, now publication Nos. WO96/00050 and WO96/06581, to which reference is directed.

## Claims

1. A dosage form useful in ophthalmic treatment comprising a jet or stream of droplets of treatment fluid, each droplet having an ophthalmologically active compound in suspension or solution.

2. A dosage form according to Claim 1 wherein the jet or each droplet has the active compound in aqueous suspension or solution.

3. A dosage form according to Claim 1 or Claim 2 wherein the jet or each droplet is of a size sufficient to sustain its momentum in transmission from a delivery device to a target site.

4. A dosage form according to Claim 3 wherein the jet or each droplet is of a size.sufficient to sustain momentum along a substantially horizontal path 5 cms in length from a discharge velocity of up to 25 m/sec from the delivery device.

5. A dosage form according to any preceding Claim wherein the jet or each droplet has a diameter in the range 100 to 800 µm.

6. A dosage form according to Claim 5 wherein the jet or each droplet has a diameter in the range 200 to 400 µm.

7. A dosage form according to any preceding Claim in which the total volume of treatment fluid does not exceed 10 µl.

8. A dosage form according to Claim 7 in which the total volume of treatment fluid is in the range 3 to 8 µl.

9. A method of ophthalmic treatment comprising delivering to an eye a dosage form according to any preceding Claim.

10. A method according to Claim 9 wherein the eye is a human eye.

11. A method according to Claim 9 or Claim 10 wherein the dosage form is directed at a particular site in the eye.

12. A method of increasing the ocular bioavailability of ophthalmologically active compound, wherein the compound is provided in suspension or solution in a body of ophthalmic treatment liquid in a dosage form comprising the liquid as a jet and/or stream of droplets, the jet and/or droplets having a mean diameter in the range 20 µm to 1000 µm.

13. A method according to Claim 12 wherein the mean diameter of the jet and/or droplets is in the range 100 µm to 800 µm, preferably 200 µm to 400 µm.

14. A method according to Claim 12 or Claim 13 wherein the total volume of treatment liquid in the dosage form does not exceed 10 µl.

15. A method according to Claim 14 wherein the total volume of treatment liquid in the dosage form is in the range 3 µl to 8 µl.